# EUROPÄISCHE PATENTSCHRIFT

(11) **EP 1 398 556 B1**
(45) Veröffentlichungstag und Bekanntmachung des Hinweises auf die Patenterteilung: **15.08.2007**
(21) Anmeldenummer: 03019860.0
(22) Anmeldetag: 01.09.2003
(51) Int. Cl.: F16L 33/22

(54) **Verbindung**
Coupling
Raccord

(30) Priorität: 11.09.2002 DE 10242096; 24.03.2003 DE 10313063
(43) Veröffentlichungstag der Anmeldung: 17.03.2004
(73) Patentinhaber: Dürr Systems GmbH, 70435 Stuttgart (DE)
(72) Erfinder: Martin, Herbert, 71384 Weinstadt (DE); Collmer, Andreas, 71665 Vaihingen/Enz (DE); Schwager, Werner, 71642 Ludwigsburg (DE); Stiegler, Martin, 71711 Steinheim (DE); Michelfelder, Manfred, 71711 Steinheim (DE)
(74) Vertreter: Heusler, Wolfgang

(56) Entgegenhaltungen:
- DE-A- 10 026 464
- DE-B- 1 165 361
- GB-A- 572 001
- US-A- 1 448 615
- US-A- 2 449 916
- US-A- 2 460 653
- US-A- 2 464 416

## Beschreibung

Die Erfindung betrifft eine Schlauch- oder Rohrverbindung mit einem Verbindungselement zum Anschluss einer rohr- oder schlauchartigen Leitung, insbesondere für den Einsatz in einer Beschichtungsanlage, gemäß dem Oberbegriff des Anspruchs 1.

Aus DE 100 26 464 A1 ist ein Schlauch-Schnellverbinder bekannt, der es ermöglicht, mittels einer Schraubverbindung einfach und schnell eine Verbindung zu einem Schlauchende herzustellen. Hierzu weist der bekannte Schlauch-Schnellverbinder einen hohlzylindrischen Anschlussstutzen auf, in den das Schlauchende eingeführt wird, wobei der Außendurchmesser des Schlauchs dem Innendurchmesser des Anschlussstutzens entspricht. An seiner Innenseite weist der hohlzylindrische Anschlussstutzen einen umlaufenden Absatz auf, an dem im montierten Zustand die Stirnfläche des Schlauchendes axial anliegt, wobei sich der Innendurchmesser des Anschlussstutzens zu dem Absatz hin verjüngt, wodurch eine Dicht- und Klemmwirkung auf das Schlauchende ausgeübt wird. Darüber hinaus weist der bekannte Schlauch-Schnellverbinder eine Überwurfmutter auf, die mittels einer Schraubverbindung unverlierbar an dem Anschlussstutzen befestigt wird und beim Aufschrauben eine auf der Mantelfläche des Schlauchs aufliegende Klemmhülse axial in Richtung des Anschlussstutzens verschiebt, wobei die Klemmhülse gegen eine Auflaufschräge stößt, welche die Klemmhülse radial gegen die Mantelfläche des Schlauchs presst und diesen dadurch in axialer Richtung fixiert. Der Schlauch wird hierbei also in dem hohlzylindrischen Anschlussstutzen gehalten und mündet schließlich in einen Durchtrittskanal mit einem kleineren Innendurchmesser.

Nachteilig an diesem bekannten Schlauch-Schnellverbinder ist zunächst die Tatsache, dass der Übergang zwischen dem Schlauch und dem Durchtrittskanal nicht molchbar ist, was insbesondere bei einem Einsatz in einer Beschichtungsanlage wichtig wäre.

Aus GB 572 001 A ist eine Schlauch- oder Rohrverbindung bekannt, die jedoch nicht molchbar ist, da ein Molch an dem Übergang zwischen dem Anschlussstutzen und der rohr- oder schlauchartigen Leitung anstoßen würde, da an diesem Übergang immer unvermeidbare Querschnittssprünge auftreten.

US 1 448 615 A und US 2 449 916 A offenbaren eine Schlauch- oder Rohrverbindung gemäss dem Obergergriff des Anspruchs 1.

Weiterhin ist zum Stand der Technik auf US 2 464 416 A, US 2 460 653 A und DE 11 65 361 B hinzuweisen; wobei die vorstehend erwähnten Patente lediglich den allgemeinen technologischen Hintergrund der Erfindung betreffen.

Der Erfindung liegt somit die Aufgabe zugrunde, eine Schlauch-oder Rohrverbindung zu schaffen, die den Einsatz eines Molchs ermöglicht.

Die Aufgabe wird, ausgehend von dem eingangs beschriebenen bekannten Schlauch-Schnellverbinder gemäß dem Oberbegriff des Anspruchs 1, durch die kennzeichnenden Merkmale des Anspruchs 1 gelöst.

Die Erfindung umfasst die allgemeine technische Lehre, den Übergang von dem Anschlussstutzen auf die rohr- oder schlauchartige Leitung molchbar zu gestalten. Dies wird vorzugsweise dadurch erreicht, dass der Übergang von dem Rohr bzw. Schlauch auf den Anschlussstutzen spaltfrei und absatzlos erfolgt, wobei der Anschlussstutzen und das Rohr bzw. die Leitung vorzugsweise denselben Innendurchmesser aufweisen.

Weiterhin ist vorgesehen, dass der Übergang von dem Anschlussstutzen auf die rohr- oder schlauchartige Leitung im Wesentlichen totraumfrei ist. Dies ist insbesondere bei einem Einsatz in einer Beschichtungsanlage vorteilhaft, da sich auf diese Weise an dem Übergang von dem Anschlussstutzen auf die Leitung keine Beschichtungsmittelreste sammeln können.

Vorzugsweise liegt die rohr- oder schlauchartige Leitung im montierten Zustand mit einer axialen Überlappung an der äußeren Mantelfläche des Anschlussstutzens an, um eine radial ausgerichtete Dichtkraft aufzunehmen.

Hierbei wird die rohr- oder schlauchartige Leitung außen auf den Anschlussstutzen aufgesteckt, so dass die äußere Mantelfläche des Anschlussstutzens an der Innenseite der rohr- oder schlauchartigen Leitung anliegt. Dies ist vorteilhaft, da so von außen eine radial wirkende Dichtkraft auf die äußere Mantelfläche der rohr- bzw. schlauchartigen Leitung aufgebracht werden kann, wobei der radial innenliegende Anschlussstutzen als Gegenlager dient. Die radial wirkende Dichtkraft kann hierbei also sehr große Werte annehmen, ohne die Leitung zu deformieren, da diese von dem Anschlussstutzen in Form gehalten wird.

In einer bevorzugten Ausführungsform des erfindungsgemäßen Verbindungselementes weist der Anschlussstutzen an seiner Außenseite einen in Längsrichtung umlaufenden Anschlag für die Stirnfläche der auf den Anschlussstutzen aufgesteckten Leitung auf, um eine umlaufende Dichtfläche zu bilden und eine axial ausgerichtete Dichtkraft aufzunehmen. Hierbei wird die rohr- oder schlauchartige Leitung also vorzugsweise außen auf den Anschlussstutzen aufgesteckt und dabei in axialer Richtung so weit geschoben, bis die Stirnfläche der Leitung axial an dem Anschlag anliegt und dadurch eine umlaufende Dichtfläche bildet.

Vorzugsweise ist ein hülsenförmiges Befestigungselement zur axialen Fixierung der Leitung an dem Anschlussstutzen vorgesehen, wobei das Befestigungselement zum Festklemmen der Leitung axial beweglich ist. Im montiertem Zustand liegt das hülsenförmige Befestigungselement vorzugsweise außen auf der Mantelfläche der auf den Anschlussstutzen aufgesteckten Leitung auf. Hierbei erfolgt das Festklemmen der Leitung an dem Anschlussstutzen also vorzugsweise durch eine Axialverschiebung des hülsenförmigen Befestigungselements.

In einer bevorzugten Ausführungsform der Erfindung weist das hülsenförmige Befestigungselement an seiner Innenseite einen Mitnahmedorn auf, der im montierten Zustand in die Mantelfläche der schlauchartigen Leitung eingreift, um die Leitung bei einer Axialverschiebung mitzunehmen. Eine Verschiebung des hülsenförmigen Befestigungselementes in axialer Richtung führt hierbei also zu einer entsprechenden axialen Verschiebung der schlauchartigen Leitung.

In einer vorteilhaften Variante der Erfindung trägt der Anschlussstutzen an seiner Außenseite ein Außengewinde, in das ein entsprechend angepasstes Innengewinde einer Überwurfmutter eingreift, wobei die Überwurfmutter vorzugsweise unverlierbar an dem Anschlussstutzen angebracht ist.

Die Überwurfmutter ermöglicht vorzugsweise eine Schraubbefestigung der rohr- bzw. schlauchartigen Leitung an dem erfindungsgemäßen Verbindungselement. Hierzu ist die Überwurfmutter vorzugsweise über einen Mitnehmer axial mit dem Befestigungselement gekoppelt, wobei der Mitnehmer das Befestigungselement beim Aufschrauben der Überwurfmutter in Richtung des Anschlussstutzens verschiebt. Ein Aufschrauben der Überwurfmutter auf den Anschlussstutzen führt hierbei also dazu, dass das Befestigungselement in axialer Richtung zum Anschlussstutzen hin verschoben wird und dabei die Leitung an dem Anschlussstutzen festklemmt. Diese Klemmwirkung bei der Axialverschiebung des hülsenförmigen Befestigungselementes wird vorzugsweise durch Auflaufschrägen erreicht, die beispielsweise an dem Anschlussstutzen angeordnet sein können. Die Auflaufschrägen führen hierbei zu einer Umsetzung der Axialbewegung des hülsenförmigen Befestigungselementes in eine zumindest teilweise radial ausgerichtete Klemmbewegung. Vorzugsweise sind sowohl an dem Anschlussstutzen als auch an dem hülsenförmigen Befestigungselement Auflaufschrägen angeordnet, die ineinander greifen und eine gleichmäßige und stufenlose Umsetzung der Axialbewegung in die radiale Klemmbewegung ermöglichen. Darüber hinaus können auch leitungsseitig an dem Befestigungselement sowie an der Überwurfmutter entsprechende Auflaufschrägen angeordnet sein.

In einer Variante der Erfindung ist die mit dem hülsenförmigen Befestigungselement zusammenwirkende Auflaufschräge nicht direkt an dem Anschlussstutzen angeordnet, sondern an einer separaten Klemmhülse, die auf den Anschlussstutzen aufgesteckt ist. Dies ist vorteilhaft, weil die Herstellung der Auflaufschräge an dem Anschlussstutzen fertigungstechnisch schwieriger ist als bei der separaten Klemmhülse. Im montierten Zustand ist die Klemmhülse vorzugsweise durch eine Presspassung mit dem Anschlussstutzen des erfindungsgemäßen Verbindungselements verbunden und dadurch in axialer Richtung fixiert. Es ist jedoch alternativ auch möglich, dass die separate Klemmhülse durch eine Schraubverbindung mit dem Anschlussstutzen verbunden ist.

Weiterhin ist in einer Variante der Erfindung vorgesehen, dass der Anschlussstutzen im Bereich der axialen Überlappung mit der schlauchartigen Leitung eine profilierte Oberfläche aufweist, um eine formschlüssige Verbindung mit der schlauchartigen Leitung zu bilden und dadurch größere axiale Haltekräfte zu erlauben. Beispielsweise kann die Oberfläche des Anschlussstutzens umlaufende Rillen aufweisen, die sich im montierten Zustand in das Wandungsmaterial der schlauchartigen Leitung einarbeiten und dadurch einen Formschluss bilden. Anstelle von Rillen kann der Anschlussstutzen im Bereich der axialen Überlappung mit der schlauchartigen Leitung jedoch auch andersartige Erhebungen oder Vertiefungen aufweisen, um die axiale Haltekraft der Verbindung zu erhöhen.

Schließlich ist in einer Variante der Erfindung vorgesehen, dass die Überwurfmutter an ihrer der Leitung zugewandten Seite eine Mündungsöffnung aufweist, die sich trompetenförmig erweitert, um ein Abknicken der schlauchartigen Leitung an:der Mündungsöffnung zu verhindern. Die trompetenförmige Erweiterung der Mündungsöffnung führt bei einem seitlichen Abknicken der schlauchartigen Leitung zu einem relativ großen Krümmungsradius an der Mündungsöffnung der Überwurfmutter, so dass relativ große Radialkräfte aufgenommen werden können, ohne dass die schlauchartige Leitung an der Knickstelle abreißt. Der im Rahmen der Erfindung verwendete Begriff einer trompetenförmigen Erweiterung der Mündungsöffnung der Überwurfmutter ist allgemein zu verstehen und nicht auf bestimmte geometrische Konturverläufe beschränkt. Entscheidend für die erfindungsgemäße Funktion ist lediglich, dass sich die Mündungsöffnung der Überwurfmutter im Wesentlichen knickfrei erweitert.

Andere vorteilhafte Weiterbildungen der Erfindung sind in den Unteransprüchen gekennzeichnet oder werden nachstehend zusammen mit der Beschreibung des bevorzugten Ausführungsbeispiels der Erfindung anhand der Figuren näher erläutert.
Es zeigen:
- Figur 1: eine Seitenansicht eines erfindungsgemäßen Verbindungselementes,
- Figur 2: eine Querschnittsansicht des Verbindungselementes aus Figur 1,
- Figur 3: eine vergrößerte Querschnittsansicht eines Ausschnitts aus Figur 2,
- Figur 4: eine Seitenansicht des hülsenförmigen Befestigungselements des Verbindungselements aus den Figuren 1 und 2,
- Figur 5: eine Querschnittsansicht eines anderen Ausführungsbeispiels eines erfindungsgemäßen Verbindungselements mit einer aufgesetzten Überwurfmutter,
- Figur 6: eine vergrößerte Querschnittsansicht des Verbindungselements aus Figur 5 ohne die Überwurfmutter sowie
- Figur 7: eine Klemmhülse des Verbindungselements aus den Figuren 5 und 6.

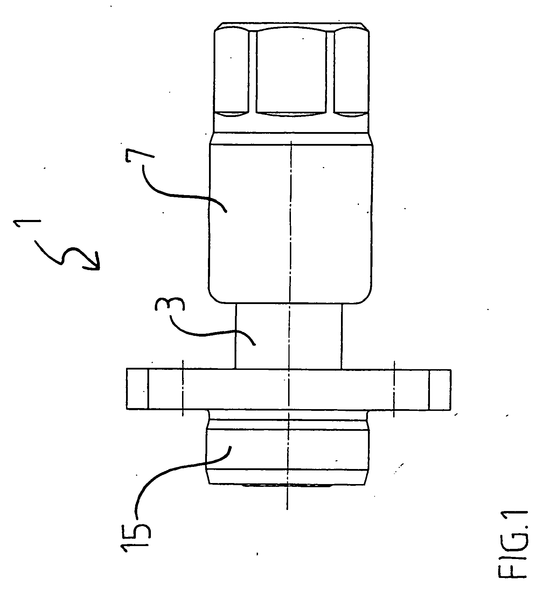

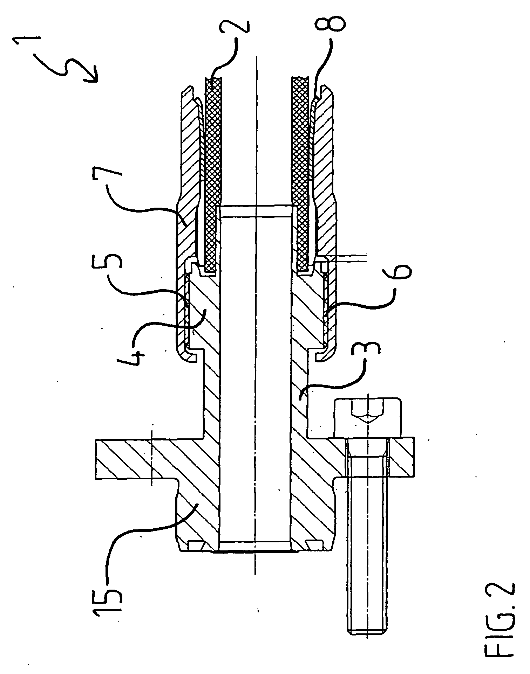

Die Figuren 1 bis 4 zeigen ein Verbindungselement 1, das in einer Lackieranlage eingesetzt wird, um einen flexiblen Schlauch 2 an einem Anschlussstutzen 3 zu befestigen, wobei der Anschlussstutzen 3 im Wesentlichen hohlzylindrisch ausgeführt ist und den gleichen Innendurchmesser aufweist wie der Schlauch 2, wie insbesondere aus Figur 2 ersichtlich ist. Dies ist beim Einsatz in einer Lackieranlage wichtig, damit der Übergang von dem Schlauch 2 auf den Anschlussstutzen 3 molchbar ist, um das Innere des Schlauchs 2 und des Anschlussstutzens 3 von Farbresten zu reinigen.

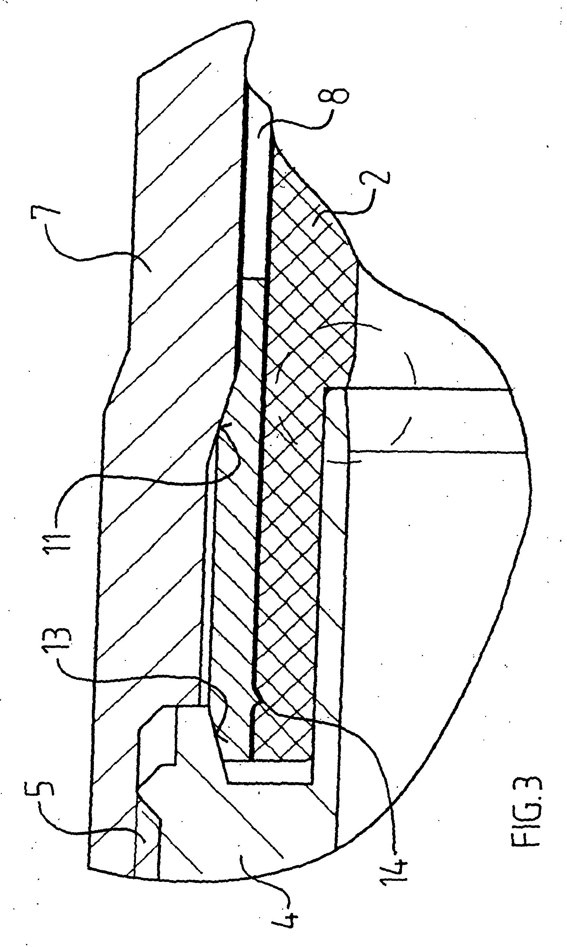

Aus der Detaildarstellung in Figur 3 ist weiterhin ersichtlich, dass der Anschlussstutzen 3 und der Schlauch 2 an dem Übergang jeweils eine Fase aufweisen, wodurch ein Anstoßen eines Molchs beim Durchgang verhindert wird.

An der Mantelfläche des Anschlussstutzens 3 ist ein umlaufender Bund 4 mit einer ebenfalls zylindrischen Mantelfläche angeformt, der ein Außengewinde 5 trägt.

Auf das Außengewinde 5 des umlaufenden Bundes 4 wird ein Innengewinde 6 einer Überwurfmutter 7 aufgeschraubt, wobei die Überwurfmutter 7 eine einfache und schnelle Schraubfixierung des Schlauchs 2 an dem Anschlussstutzens 3 ermöglicht, wie noch detailliert beschrieben wird.

An der dem Anschlussstutzen 3 zugewandten Stirnseite ist die Wandung der Überwurfmutter 7 radial nach innen umgebogen und greift hierbei hinter den Bund 4, so dass die Überwurfmutter 7 unverlierbar an dem Anschlussstutzen 3 befestigt ist.

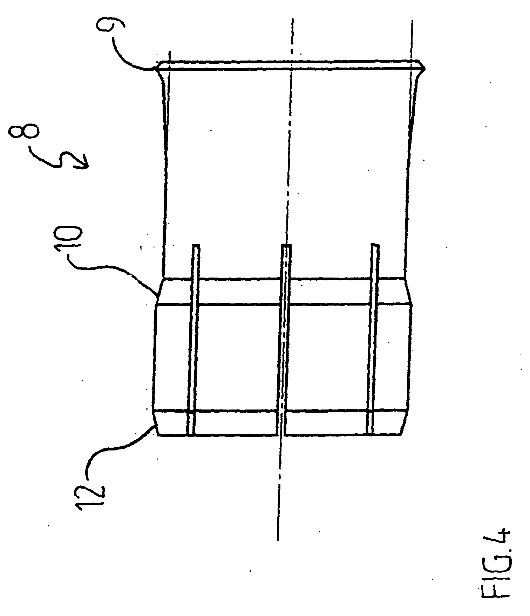

Zwischen dem Schlauch 2 und der Überwurfmutter 7 ist hierbei ein hülsenförmiges Befestigungselement 8 angeordnet, das in Figur 4 in einer Seitenansicht wiedergegeben ist. Das hülsenförmige Befestigungselement 8 hat die Aufgabe, die beim Aufschrauben der Überwurfmutter 7 entstehende Axialbewegung der Überwurfmutter 7 in Richtung des Anschlussstutzens 3 in eine Klemmbewegung umzuwandeln, die den Schlauch 2 auf dem Anschlussstutzen 3 fixiert.

Hierzu weist das hülsenförmige Befestigungselement 8 an seinem dem Schlauch 2 zugewandten axialen Ende einen radial hervorstehenden Vorsprung 9 auf, der in eine entsprechende Aussparung an der Innenseite der Überwurfmutter 7 eingreift, wodurch das hülsenförmige Befestigungselement 8 bei einem Losschrauben der Überwurfmutter 7 von dem Anschlussstutzen 3 in axialer Richtung mitgenommen wird, um die Klemmverbindung zu lösen.

Weiterhin weist das hülsenförmige Befestigungselement 8 in seinem mittleren Bereich eine Auflaufschräge 10 auf, die zu einer entsprechenden Auflaufschräge 11 an der Innenseite der Überwurfmutter 7 korrespondiert, so dass die Überwurfmutter 7 das hülsenförmige Befestigungselement 8 beim Aufschrauben auf den Anschlussstutzen 3 zunächst in axialer Richtung zu dem Anschlussstutzen 3 hin verschiebt und das hülsenförmige Befestigungselement 8 darüber hinaus in radialer Richtung von außen auf den Anschlussstutzen 3 auftrifft.

Weiterhin weist das hülsenförmige Befestigungselement 8 an seinem dem Anschlussstutzen 3 zugewandten Ende eine weitere Auflaufschräge 12 auf, die zu einer Auflaufschräge 13 korrespondiert, die an dem Umfangsrand des Bundes 4 angeformt. Das Ineinandergreifen der beiden Auflaufschrägen 12, 13 führt beim Aufschrauben der Überwurfmutter 7 auf den Anschlussstutzen 3 dazu, dass das hülsenförmige Befestigungselement in radialer Richtung auf die äußere Mantelfläche des Schlauchs aufgepresst wird.

Ferner weist das hülsenförmige Befestigungselement 8 an seiner Innenseite einen radial vorstehenden Mitnahmedorn 14 auf, der in die äußere Mantelfläche des Schlauchs 2 eingreift und den Schlauch 2 bei einer Axialbewegung des hülsenförmigen Befestigungselementes 8 in axialer Richtung mitnimmt.

Von Bedeutung ist weiterhin, dass der Schlauch an seiner Innenseite einen umlaufenden Absatz aufweist, an dem die Stirnfläche des Anschlussstutzens 3 axial anliegt, wodurch axial ausgerichtete Dichtkräfte aufgenommen werden können. Die radiale Erstreckung dieses Absatzes ist hierbei gleich der Wandstärke des Anschlüssstutzens 3 an seinem freien Ende, so dass der Schlauch 2 an dem Übergang zu dem Anschlussstutzen 3 absatzlos und spaltfrei übergeht. Dies ermöglicht vorteilhaft den Einsatz eines Molchs zur Reinigung des Schlauchs 2 und des Anschlussstutzens 3.

Der Anschlussstutzen 3 weist an seiner Außenseite ebenfalls einen umlaufenden Absatz auf, der einen axialen Anschlag für die Stirnfläche des Schlauchs 2 bildet.

Auf der dem Anschlussstutzen 3 abgewandten Seite weist das Verbindungselement 1 einen Anschlussflansch 15 auf.

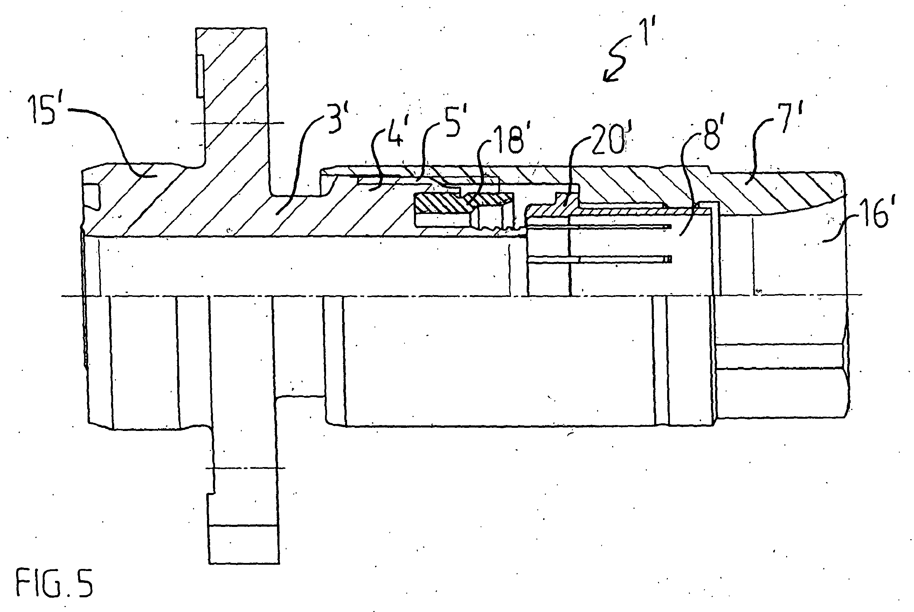

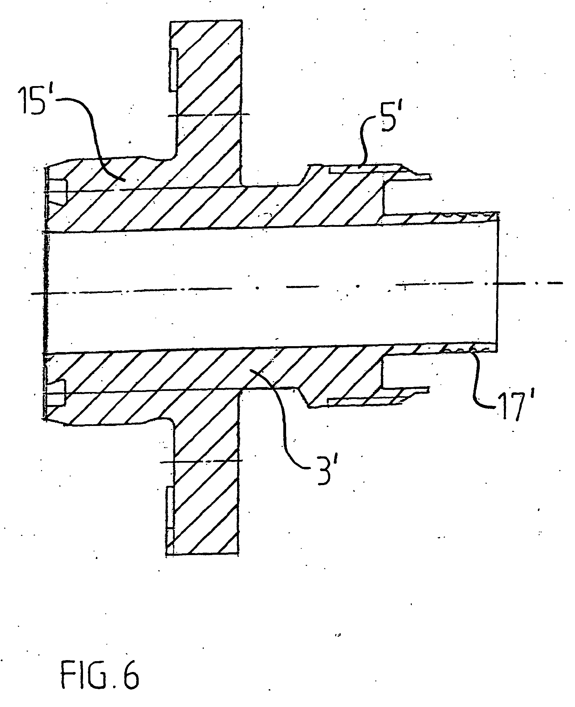

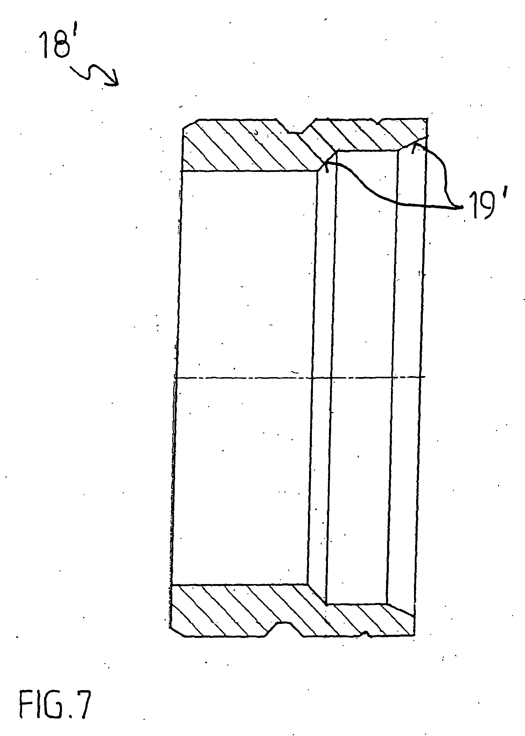

Das in den Figuren 5 bis 7 dargestellte Ausführungsbeispiel eines erfindungsgemäßen Verbindungselements 1' stimmt weitgehend mit dem vorstehend beschriebenen Ausführungsbeispiel überein, so dass im Folgenden zur Vermeidung von Wiederholungen auf die vorstehende Beschreibung verwiesen wird und für entsprechende Bauteile dieselben Bezugszeichen verwendet werden, die zur Vermeidung von Verwechslungen lediglich durch ein Apostroph gekennzeichnet sind.

Ein Besonderheit dieses Ausführungsbeispiels besteht darin, dass die Überwurfmutter 7' eine Mündungsöffnung 16' aufweist, die sich trompetenförmig erweitert. Bei einem seitlichen Abknicken der schlauchartigen Leitung wird dadurch ein Leitungsbruch weitgehend verhindert, da der Krümmungsradius an der Knickstelle durch den Oberflächenverlauf der Mündungsöffnung 16' vorgegeben ist.

Eine weitere Besonderheit dieses Ausführungsbeispiels besteht darin, dass der Anschlussstutzen 3' in dem Bereich der axialen Überlappung mit der schlauchartigen Leitung umlaufende Rillen 17' aufweist, die sich in montiertem Zustand in das Wandungsmaterial der schlauchartigen Leitung einarbeiten und dadurch eine formschlüssige Verbindung zwischen dem Anschlussstutzen 3' und der.schlauchartigen Leitung bilden, wodurch die axialen Haltekräfte erhöht werden.

Darüber hinaus weist das Verbindungselement 1' in dieser Variante eine Klemmhülse 18' auf, an deren Innenwandung eine Auflaufschräge 19' ausgebildet ist. Die Klemmhülse 18' ist hierbei auf den Anschlussstutzen 3' aufgepresst, wobei der Anschlussstutzen 3' unter die Klemmhülse 18' eine Presspassung bilden, so dass die Klemmhülse 18' im montierten Zustand axial fixiert ist. Die Anordnung der Auflaufschräge 19' an der separaten Klemmhülse 18' ist fertigungstechnisch wesentlich einfacher als die Anbringung der Auflaufschräge 13' direkt an dem Anschlussstutzen 3, wie bei dem Ausführungsbeispiel entsprechend den Figuren 1 bis 4.

Schließlich weist das hülsenförmige Befestigungselement 8' hierbei einen radial vorstehenden, umlaufenden Bund 20' auf, der an einem entsprechenden Absatz an der Innenwand der Überwurfmutter 7' anliegt, so dass die Überwurfmutter 7' das hülsenförmige Befestigungselement 8' in axialer Richtung mitnimmt.

## Patentansprüche

1. Schlauch- oder Rohrverbindung, insbesondere für den Einsatz in einer Beschichtungsanlage, mit
a) einer rohr- oder schlauchartigen Leitung (2),
b) einem Verbindungselement (1, 1') zum Anschluss der Leitung (2) mit
c) einem Anschlussstutzen (3, 3') zum Aufstecken der Leitung (2) und
d) einem Befestigungselement (8, 8') zur formschlüssigen und/oder reibschlüssigen Axialfixierung der Leitung (2) an dem Anschlussstutzen (3, 3'),
e) wobei der Anschlussstutzen (3, 3') und die Leitung (2) im wesentlichen den gleichen Innendurchmesser aufweisen,
f) einem molchbaren Übergang von dem Anschlussstutzen (3, 3') auf die Leitung (2),
**dadurch gekennzeichnet daß** der Anschlussstutzen (3, 3') und die Leitung (2)
g) an ihrer Innenwand an dem Übergang jeweils eine Fase aufweisen und
h) einen im Wesentlichen totraumfreien Übergang von dem Anschlussstutzen (3, 3') auf die Leitung (2) aufweisen.

2. Schlauch- oder Rohrverbindung nach Anspruch 1, **dadurch gekennzeichnet, dass** die Leitung (2) im montierten Zustand mit einer axialen Überlappung an der inneren oder äußeren Mantelfläche des Anschlussstutzens (3, 3') anliegt, um eine radial ausgerichtete Dichtkraft aufzunehmen.

3. Schlauch- oder Rohrverbindung nach mindestens einem der vorhergehenden Ansprüche, **dadurch gekennzeichnet, dass** der Anschlussstutzen (3, 3') an seiner Außenseite einen bezüglich seiner Längsachse umlaufenden Anschlag für die Stirnfläche der auf den Anschlussstutzen (3, 3') aufgesteckten Leitung (2) aufweist, um eine umlaufende Dichtfläche zu bilden und eine axial ausgerichtete Dichtkraft aufzunehmen.

4. Schlauch- oder Rohrverbindung nach mindestens einem der vorhergehenden Ansprüche, **dadurch gekennzeichnet, dass** das Befestigungselement (8, 8') hülsenförmig ausgebildet und zum Festklemmen der Leitung (2) axial beweglich ist, wobei das Befestigungselement (8, 8') im montierten Zustand außen auf der Mantelfläche der auf den Anschlussstutzen (3, 3') aufgesteckten Leitung (2) aufliegt.

5. Schlauch- oder Rohrverbindung nach Anspruch 4, **dadurch gekennzeichnet, dass** an der Innenseite des hülsenförmigen Befestigungselements (8, 8') ein Mitnahmedorn (14) angebracht ist, der im montierten Zustand in die Mantelfläche der Leitung (2) eingreift, um die Leitung (2) bei einer Axialverschiebung mitzunehmen.

6. Schlauch- oder Rohrverbindung nach mindestens einem der vorhergehenden Ansprüche, **dadurch gekennzeichnet, dass** der Anschlussstutzen (3, 3') an seiner Außenseite ein Außengewinde (5, 5') trägt, in das ein entsprechend angepasstes Innengewinde (6, 6') einer Überwurfmutter (7, 7') eingreift.

7. Schlauch- oder Rohrverbindung nach Anspruch 6, **dadurch gekennzeichnet, dass** die Überwurfmutter (7, 7') über einen Mitnehmer axial mit dem Befestigungselement (8, 8') gekoppelt ist, wobei der Mitnehmer das Befestigungselement (8, 8') beim Aufschrauben und/oder beim Abschrauben der Überwurfmutter (7, 7') axial mitnimmt.

8. Schlauch- oder Rohrverbindung nach Anspruch 6 und/oder Anspruch 7, **dadurch gekennzeichnet, dass** die Überwurfmutter (7, 7') unverlierbar an dem Anschlussstutzen (3, 3') angebracht ist.

9. Schlauch- oder Rohrverbindung nach mindestens einem der vorhergehenden Ansprüche, **dadurch gekennzeichnet, dass** der Anschlussstutzen (3, 3') an seiner Außenseite eine erste Auflaufschräge (13) für das Befestigungselement (8, 8') aufweist, die das Befestigungselement (8, 8') bei einer Axialbewegung in Richtung des Anschlussstutzens (3, 3') radial auf die Leitung (2) presst.

10. Schlauch- oder Rohrverbindung nach mindestens einem der vorhergehenden Ansprüche, **dadurch gekennzeichnet, dass** das Befestigungselement (8, 8') stutzenseitig eine zweite Auflaufschräge (12) aufweist, die das Befestigungselement (8, 8') bei einer Axialbewegung in Richtung des Anschlussstutzens (3, 3') radial auf die Leitung (2) presst.

11. Schlauch- oder Rohrverbindung nach mindestens einem der Ansprüche 6 bis 8, **dadurch gekennzeichnet, dass** die Überwurfmutter (7, 7') an ihrer Innenseite eine dritte Auflaufschräge (11) aufweist, die das Befestigungselement (8, 8') beim Aufschrauben der Überwurfmutter radial auf die Leitung (2) presst.

12. Schlauch- oder Rohrverbindung nach mindestens einem der Ansprüche 6 bis 8 oder 11, **dadurch gekennzeichnet, dass** das Befestigungselement (8, 8') leitungsseitig eine vierte Auflaufschräge (10) aufweist, die das Befestigungselement (8, 8') beim Aufschrauben der Überwurfmutter (7, 7') radial auf die Leitung (2) presst.

13. Schlauch- oder Rohrverbindung nach mindestens einem der vorhergehenden Ansprüche, **dadurch gekennzeichnet, dass** der Anschlussstutzen (3') im Bereich der axialen Überlappung mit der schlauchartigen Leitung eine profilierte Oberfläche (17') aufweist, um eine formschlüssige Verbindung mit der schlauchartigen Leitung (2) zu bilden.

14. Schlauch- oder Rohrverbindung nach mindestens einem der vorhergehenden Ansprüche, **dadurch gekennzeichnet, dass** auf dem Anschlussstutzen (3') eine Klemmhülse (18') angeordnet ist, wobei die Klemmhülse (18') an ihrer Innenseite eine Auflaufschräge (19') für das hülsenförmige Befestigungselement (8') aufweist, die das Befestigungselement (8') bei einer Axialbewegung in Richtung des Anschlussstutzens (3') radial auf die Leitung (2) drückt.

15. Schlauch- oder Rohrverbindung nach mindestens einem der Ansprüche 6 bis 8, 11 oder 12, **dadurch gekennzeichnet, dass** die Überwurfmutter (7') an ihrer der Leitung (2) zugewandten Seite eine Mündungsöffnung (16') aufweist, die sich trompetenförmig erweitert.

## Claims

1. A tube connection or pipe connection, especially for the use in a coating system, with
a) a pipe-like or tube-like line (2),
b) a connection element (1, 1') for the connection to the line (2) with
c) a connecting piece (3, 3') onto which the line (2) is slipped, and
d) a fastening element (8, 8') for positive locking and/or frictionally engaged axial attachment of the line (2) onto the connecting piece (3, 3'),
e) whereby the connecting piece (3, 3') and the line (2) have substantially the same inner diameter,
f) a piggable junction from the connecting piece (3, 3') to the line (2),
**characterised in that**
the connecting piece (3, 3') and the line (2)
g) each has a bevel on their inner wall at the junction and
h) have a transition with substantially no dead space from the connecting piece (3, 3') to the line (2).

2. A tube connection or pipe connection according to claim 1, **characterised in that** the line (2) in the assembled state abuts against the inner or outer lateral surface of the connecting piece (3, 3') with an axial overlap, in order to accept a radially directed sealing force.

3. A tube connection or pipe connection according to at least one of the preceding claims, **characterised in that** the connecting piece (3, 3') has on its exterior a circumferential stop, relative to the longitudinal axis, for the end face of the line (2), which is slipped onto the connecting piece (3, 3') in order to form a circumferential sealing surface and to accept an axially directed sealing force.

4. A tube connection or pipe connection according to at least one of the preceding claims, **characterised in that** the fastening element (8, 8') is formed in the shape of a sleeve and can be moved axially to clamp the line (2), whereby the fastening element (8, 8') in the assembled state abuts on the outside against the lateral surface of the line (2) slipped onto the connecting piece (3,3').

5. A tube connection or pipe connection according to claim 4, **characterised in that** a carrier peg (14) is installed on the inside of the sleeve-shaped fastening element (8, 8'), and in the assembled state engages into the lateral surface of the line (2), to move the line (2) in the event of an axial movement.

6. A tube connection or pipe connection according to at least one of the preceding claims, **characterised in that** the connecting piece (3, 3') carries on its exterior an external screwthread (5, 5'), in which a correspondingly adapted internal screwthread (6, 6') of a union nut (7, 7') engages.

7. A tube connection or pipe connection according to claim 6, **characterised in that** the union nut (7, 7') is connected axially to the fastening element (8, 8') through an activator, whereby the activator takes the fastening element (8, 8') along axially during screwing-on and/or unscrewing of the union nut (7, 7').

8. A tube connection or pipe connection according to claim 6 and/or claim 7, **characterised in that** the union nut (7, 7') is permanently mounted on the connecting piece (3, 3').

9. A tube connection or pipe connection according to at least one of the preceding claims, **characterised in that** the connecting piece (3, 3') on its exterior has a first engagement chamfer (13) for the fastening element (8, 8'), which presses the fastening element (8, 8') radially onto the line (2) during an axial movement in the direction of the connecting piece (3, 3').

10. A tube connection or pipe connection according to at least one of the preceding claims, **characterised in that** the fastening element (8, 8') on its connection side has a second engagement chamfer (12), which presses the fastening element (8, 8') radially onto the line (2) during an axial movement in the direction of the connecting piece (3, 3').

11. A tube connection or pipe connection according to at least one of claims 6 through 8, **characterised in that** the union nut (7, 7') has, on its inside, a third engagement chamfer (11), which presses the fastening element (8,8') radially onto the line (2) when the union nut is being screwed on.

12. A tube connection or pipe connection according to at least one of the preceding claims, **characterised in that** the fastening element (8, 8') on its line side has a fourth engagement chamfer (10), which presses the fastening element (8, 8') radially onto the line (2) when the connecting piece (7, 7') is screwed on.

13. A tube connection or pipe connection according to at least one of the preceding claims, **characterised in that** the connecting piece (3') in the region of the axial overlap with the tube-like line has a profiled surface (17'), in order to form a positive locking connection with the tube-like line (2).

14. A tube connection or pipe connection according to at least one of the preceding claims, **characterised in that** the a clamping sleeve (18') is arranged on the connecting piece (3'), whereby the clamping sleeve has, on its inside, an engagement chamfer (19') for the sleeve-like fastening element (8'), which presses the fastening element (8') radially onto the line (2) during an axial movement in the direction of the connecting piece (3').

15. A tube connection or pipe connection according to one of the claims 6 to 8, 11 or 12, **characterised in that** the union nut (7') on its side facing toward the line (2) has an opening (16'), which widens in the shape of a trumpet.

## Revendications

1. Raccord pour tuyau flexible ou tube, notamment pour une utilisation dans une installation de revêtement, comportant
a) une conduite en tube ou en tuyau flexible (2),
b) un élément de raccordement (1, 1') pour la jonction de la conduite (2) à
c) un raccord (3, 3') pour l'emboîtement de la conduite (2) et
d) un élément de fixation (8, 8') pour la fixation axiale par coopération de formes et/ou par friction de la conduite (2) au raccord (3, 3'),
e) sachant que le raccord (3, 3') et la conduite (2) présentent pour l'essentiel le même diamètre interne,
f) une jonction pouvant être raclée du raccord (3, 3') à la conduite (2),
**caractérisé en ce que**
g) le raccord (3, 3') et la conduite (2) présentent chacun un chanfrein sur leur paroi interne, au niveau de la jonction, et
h) comportent une jonction pour l'essentiel sans espace mort du raccord (3, 3') à la conduite (2).

2. Raccord pour tuyau flexible ou tube selon la revendication 1, **caractérisé en ce que** la conduite (2) s'appuie, à l'état assemblé, avec un chevauchement axial, sur la surface latérale interne ou externe du raccord (3, 3') pour absorber une force d'étanchéité orientée dans le sens radial.

3. Raccord pour tuyau flexible ou tube selon au moins l'une des revendications précédentes, **caractérisé en ce que** le raccord (3, 3') présente, sur son côté externe, une butée circulaire par rapport à son axe longitudinal pour la surface frontale de la conduite (2) emboîtée sur le raccord (3, 3') afin de former une surface d'étanchéité circulaire et d'absorber une force d'étanchéité orientée dans le sens axial.

4. Raccord pour tuyau flexible ou tube selon au moins l'une des revendications précédentes, **caractérisé en ce que** l'élément de fixation (8, 8') est réalisé en forme de douille et peut être déplacé dans le sens axial pour bloquer la conduite (2), sachant que l'élément de fixation (8, 8') repose, à l'état assemblé, à l'extérieur, sur la surface latérale de la conduite (2) emboîtée sur le raccord (3, 3').

5. Raccord pour tuyau flexible ou tube selon la revendication 4, **caractérisé en ce qu'**est monté, sur le côté interne de l'élément de fixation en forme de douille (8, 8'), un mandrin d'entraînement (14) qui s'engrène, à l'état assemblé, dans la surface latérale de la conduite (2) afin d'entraîner la conduite (2) lors d'un déplacement axial.

6. Raccord pour tuyau flexible ou tube selon au moins l'une des revendications précédentes, **caractérisé en ce que** le raccord (3, 3') comporte, sur son côté externe, un filet externe (5, 5') dans lequel s'engrène un filet interne (6, 6'), adapté de manière correspondante, d'un écrou à chapeau (7, 7').

7. Raccord pour tuyau flexible ou tube selon la revendication 6, **caractérisé en ce que** l'écrou à chapeau (7, 7') est couplé à l'élément de fixation (8, 8') dans le sens axial par le biais d'un taquet d'entraînement, sachant que le taquet d'entraînement entraîne l'élément de fixation (8, 8') dans le sens axial lors du vissage et/ou du dévissage de l'écrou à chapeau (7, 7').

8. Raccord pour tuyau flexible ou tube selon la revendication 6 et/ou la revendication 7, **caractérisé en ce que** l'écrou à chapeau (7, 7') est monté de manière imperdable sur le raccord (3, 3').

9. Raccord pour tuyau flexible ou tube selon au moins l'une des revendications précédentes, **caractérisé en ce que** le raccord (3, 3') comporte, sur son côté externe, un premier chanfrein d'arrêt (13) pour l'élément de fixation (8, 8') qui presse l'élément de fixation (8, 8') dans le sens radial sur la conduite (2) lors d'un mouvement axial en direction du raccord (3, 3').

10. Raccord pour tuyau flexible ou tube selon au moins l'une des revendications précédentes, **caractérisé en ce que** l'élément de fixation (8, 8') comporte, du côté du raccord, un second chanfrein d'arrêt (12) qui presse l'élément de fixation (8, 8') dans le sens radial sur la conduite (2) lors d'un mouvement axial en direction du raccord (3, 3').

11. Raccord pour tuyau flexible ou tube selon au moins l'une des revendications 6 à 8, **caractérisé en ce que** l'écrou à chapeau (7, 7') comporte, sur son côté interne, un troisième chanfrein d'arrêt (11) qui presse l'élément de fixation (8, 8') dans le sens radial sur la conduite (2) lors du vissage de l'écrou à chapeau.

12. Raccord pour tuyau flexible ou tube selon au moins l'une des revendications 6 à 8 ou 11, **caractérisé en ce que** l'élément de fixation (8, 8') comporte, côté conduite, un quatrième chanfrein d'arrêt (10) qui presse l'élément de fixation (8, 8') dans le sens radial sur la conduite (2) lors du vissage de l'écrou à chapeau. (7, 7').

13. Raccord pour tuyau flexible ou tube selon au moins l'une des revendications précédentes, **caractérisé en ce que** le raccord (3') comporte, au niveau du chevauchement axial avec la conduite en tuyau flexible, une superficie moulée (17') afin de former une liaison par coopération de formes avec la conduite en tuyau flexible (2).

14. Raccord pour tuyau flexible ou tube selon au moins l'une des revendications précédentes, **caractérisé en ce que**, sur le raccord (3'), est disposée une douille de serrage (18'), sachant que la douille de serrage (18') comporte, sur son côté interne, un chanfrein d'arrêt (19') pour l'élément de fixation en forme de douille (8') qui comprime l'élément de fixation (8') dans le sens radial sur la conduite (2) lors d'un mouvement axial en direction du raccord (3').

15. Raccord pour tuyau flexible ou tube selon au moins l'une des revendications 6 à 8, 11 ou 12, **caractérisé en ce que** l'écrou à chapeau (7') comporte, sur son côté tourné vers la conduite (2), un orifice débouchant (16') qui s'élargit en forme de trompette.
